# EUROPEAN PATENT SPECIFICATION

(11) **EP 2 638 175 B1**
(45) Date of publication and mention of the grant of the patent: **17.05.2017**
(21) Application number: 10790739.6
(22) Date of filing: 09.11.2010
(51) Int. Cl.: C12Q 1/68, G01N 33/50, G01N 33/58

(54) **METHOD FOR THE SELECTION OF MODULATORS OF BDNF**
VERFAHREN ZUR AUSWAHL VON BDNF MODULATOREN
MÉTHODE POUR LE CHOIX DES MODULATEURS DE BDNF

(43) Date of publication of application: 18.09.2013
(73) Proprietor: Universita' Degli Studi di Trieste, I-34127 Trieste (IT)
(72) Inventor: TONGIORGI, Enrico, 34123 Trieste (IT); VAGHI, Valentina, 22079 Villa Guardia (IT); VICARIO, Annalisa, 33030 Moruzzo (IT); BAJ, Gabriele, 21100 Varese (IT)
(74) Representative: Zaccaro, Elisabetta
(86) International application number: PCT/EP2010/067081
(87) International publication number: WO 2012/062354

(56) References cited:
- WO-A2-2010/028651
- US-A1- 2003 149 994
- OE S ET AL: "Cytoplasmic polyadenylation element-like sequences are involved in dendritic targeting of BDNF mRNA in hippocampal neurons", FEBS LETTERS, ELSEVIER, AMSTERDAM, NL, vol. 584, no. 15, 4 August 2010 (2010-08-04), pages 3424-3430, XP027182864, ISSN: 0014-5793 [retrieved on 2010-07-08]
- AID TAMARA ET AL: "Mouse and rat BDNF gene structure and expression revisited", JOURNAL OF NEUROSCIENCE RESEARCH, WILEY-LISS, US, vol. 85, no. 3, 15 February 2007 (2007-02-15), pages 525-535, XP002587238, ISSN: 0360-4012
- DATABASE EMBL [Online] EMBL; 8 January 2007 (2007-01-08), Aid T. et al.: "Rattus norvegicus brain-derived neurotrophic factor precursor transcript variant VI mRNA", XP000002657655, Database accession no. EF125680

### SEQUENCE MISTING

<110> UNIVERSITA' DEGLI STUDI DI TRIESTE
<120> METHOD FOR THE SELECTION OF COMPOUNDS USEFUL FOR THE TREATMENT OF NEUROPSYCHIATRIC AND NEURODEGENARATIVE DISEASES
<130> 10573PTWO
<160> 14
<170> PatentIn version 3.5
<210> 1
   <211> 613
   <212> DNA
   <213> Rattus norvegicus
<400> 1
   taaagcggta gccggctggt gcaggaaagc aacaagttcc ccagcggtct tcccgcccta      60
   gcctgacaag gcgaaggttt tcttacctgg cgacagggaa atctcctgag ccgagctcat     120
   ctttgccaca gccccaggtg tgacctgagc agtgggcaaa ggagcggcgt gcaaattgga     180
   ttatttgtat gggggtactc tgaaactccc tcactttttc tgggaacttt ttgtgctagg     240
   gcgcagtgac aggcgttgag aaagctgctt caggaaacgc ccgctatata gcagggcagt     300
   tggacagtca ttggtaacct cgctcattca ttagaatcac gtaagaactc aaagggaaac     360
   gtgtctctca gaatgagggc gtttgcgtaa atctataggt ttttcaacat cgatgccagt     420
   tgctttgtct tctgtaatcg ccaaggtgga tgagagttga agcttgcgga tattgcaaag     480
   ggttattaga ttcataagtc acaccaagtg gtgggcgatc cactgagcaa agccgaactt     540
   ctcacatgat gacttcaaac aagacacatt accttccagc atctgttggg gagacgagat     600
   tttaagacac tga                                                        613
<210> 2
   <211> 205
   <212> DNA
   <213> Rattus norvegicus
<400> 2
   gctttggcaa agccatccgc acgtgacaaa acgtaaggaa gtggaagaaa ccgtctagag      60
   caatatcaag taccacttaa ttagagaata tttttttaac cttttcctcc tgctgcgccg     120
   ggtgtgtgat ccgggcgagc agagtccatt cagcaccttg gaacagagcc agcggatttg     180
   tccgaggtgg tagtacttca tccag                                           205
<210> 3
   <211> 415
   <212> DNA
   <213> Rattus norvegicus
<400> 3
   gctttggcaa agccatccgc acgtgacaaa acgtaaggaa gtggaagaaa ccgtctagag      60
   caatatcaag taccacttaa ttagagaata tttttttaac cttttcctcc tgctgcgccg     120
   ggtgtgtgat ccgggcgagc agagtccatt cagcaccttg gaacagagcc agcggatttg     180
   tccgaggtgg tagtacttca tccaggtatt cttttcctcg ctgtcaagcc aacccggtgt     240
   cgcccttaaa aagcgtcttt tccgaggttc ggctcacact gagatcgggg ctggagagag     300
   agtcagattt tggagcggag cgtttggaga gccagcccca gtttggtccc ctcattgagc     360
   tcgctgaagt tggcttccta gcggtgtagg ctggaataga ctcttggcaa gctcc          415
<210> 4
   <211> 500
   <212> DNA
   <213> Rattus norvegicus
<400> 4
   gctttggcaa agccatccgc acgtgacaaa acgtaaggaa gtggaagaaa ccgtctagag     60
   caatatcaag taccacttaa ttagagaata tttttttaac cttttcctcc tgctgcgccg    120
   ggtgtgtgat ccgggcgagc agagtccatt cagcaccttg gaacagagcc agcggatttg    180
   tccgaggtgg tagtacttca tccaggtatt cttttcctcg ctgtcaagcc aacccggtgt    240
   cgcccttaaa aagcgtcttt tccgaggttc ggctcacact gagatcgggg ctggagagag    300
   agtcagattt tggagcggag cgtttggaga gccagcccca gtttggtccc ctcattgagc    360
   tcgctgaagt tggcttccta gcggtgtagg ctggaataga ctcttggcaa gctccgggtt    420
   ggtatactgg gttaactttg ggaaatgcaa gtgtttatct ccaggatcta gccaccgggg    480
   tggtgtaagc cgcaaagaag                                                500
<210> 5
   <211> 215
   <212> DNA
   <213> Rattus norvegicus
<400> 5
   gccctcacga ttctcgctgg atagttcttt atgttttggg tgatttttgt tttttgtttg     60
   tttggttttt ttttttttta atttttaccc ctttctattt tccctccccg agagttccgg    120
   gctctggctt ggagggctcc tgctttctca agggaagggg agccgctgag actgcgctcc    180
   actccctgcc gggctggatg cttcattgag cccag                               215
<210> 6
   <211> 335
   <212> DNA
   <213> Rattus norvegicus
<400> 6
   acccactttc ccattcaccg aggagaggac tgctcgcgct gccgctcccc ccacccaccc     60
   ccggcgagct agcatgaaat ctcccagtct ctgcctagat caaatggagc ttctcactga 120
   aggcgtgcga gtattacctc cgccatgcaa tttccactat caataattta acttctttgc 180
   tgaagaacag gagtacatat cggccaccaa agactcgccc ccctccccct tttaactgaa 240
   gagaagggga aatatatagt aagagtctag aaccttgggg accggtcttc cccagagcag 300
   ctgccttgat gtttactttg acaagtagtg actga 335
<210> 7
   <211> 81
   <212> DNA
   <213> Rattus norvegicus
<400> 7
   aaaccataac cccgcacact ctgtgtagtt tcattgtgtg ttcgcttttt ctagctctgt 60
   ggtgcgggaa ggtgcgggaa g 81
<210> 8
   <211> 348
   <212> DNA
   <213> Rattus norvegicus
<400> 8
   ccaatcgaag ctcaaccgaa gagctaaata atgtctgacc ccagtgcctg gcgctggctg 60
   agctctgggt gcccgccgct gccgccgcgc cggggcgcac ccgctggctg gctgtcgcac 120
   ggtccccatt gcgcccggga ctccccggct tggagaagga aaccgcctgg ggcggcgcgc 180
   cacctccgcc tggcaggctt tgatgagacc gggttccctc agctcgccac cgctgctttg 240
   gggcagacga gaaagcgcac ggggcccagg gcagggcgca gggaccagga gcgtgacaac 300
   aatgtgactc cactgccggg gatccgagag ctttgtgtgg accctgag 348
<210> 9
   <211> 213
   <212> DNA
   <213> Rattus norvegicus
<400> 9
   cactgtcacc tgctttctag ggagtattac caaagcttct tacaagtcca aggtcaacat 60
   aactaggaac ttgggatcat tcttgtcact gggacctgaa agggtctgcg gaactccagg 120
   tcccacagct tgtatccgac cctctctgtc catccagcgc acctctttag gcatcctcca 180
   gaggaagtga aagttttgac tttcatccgg gag 213
<210> 10
   <211> 256
   <212> DNA
   <213> Rattus norvegicus
<400> 10
   gttatagagt tggatgcaag cgtaacccga aggacaactg gatgtgtgga agcaccagtt      60
   ttctccatgt gctcaggcta atcctcgtta atactcagaa cgttgagttt ctacggaatg     120
   ggttgcatgt ccatacatgt tttggtatct acccacacac tttcatgtgg tatgactgtg     180
   catcccagga gaaaggctgt ggtgtgtgtc tctgcgcctc agtggagctg aacaaacgat     240
   tgctgaaaat ggtgtc                                                     256
<210> 11
   <211> 1653
   <212> DNA
   <213> Rattus norvegicus
<400> 11
   atggaagacg ccaaaaacat aaagaaaggc ccggcgccat tctatccgct ggaagatgga      60
   accgctggag agcaactgca taaggctatg aagagatacg ccctggttcc tggaacaatt     120
   gcttttacag atgcacatat cgaggtggac atcacttacg ctgagtactt cgaaatgtcc     180
   gttcggttgg cagaagctat gaaacgatat gggctgaata caaatcacag aatcgtcgta     240
   tgcagtgaaa actctcttca attctttatg ccggtgttgg gcgcgttatt tatcggagtt     300
   gcagttgcgc ccgcgaacga catttataat gaacgtgaat tgctcaacag tatgggcatt     360
   tcgcagccta ccgtggtgtt cgtttccaaa aaggggttgc aaaaaatttt gaacgtgcaa     420
   aaaaagctcc caatcatcca aaaaattatt atcatggatt ctaaaacgga ttaccaggga     480
   tttcagtcga tgtacacgtt cgtcacatct catctacctc ccggttttaa tgaatacgat     540
   tttgtgccag agtccttcga tagggacaag acaattgcac tgatcatgaa ctcctctgga     600
   tctactggtc tgcctaaagg tgtcgctctg cctcatagaa ctgcctgcgt gagattctcg     660
   catgccagag atcctatttt tggcaatcaa atcattccgg atactgcgat tttaagtgtt     720
   gttccattcc atcacggttt tggaatgttt actacactcg gatatttgat atgtggattt     780
   cgagtcgtct taatgtatag atttgaagaa gagctgtttc tgaggagcct tcaggattac     840
   aagattcaaa gtgcgctgct ggtgccaacc ctattctcct tcttcgccaa aagcactctg     900
   attgacaaat acgatttatc taatttacac gaaattgctt ctggtggcgc tcccctctct     960
   aaggaagtcg gggaagcggt tgccaagagg ttccatctgc caggtatcag gcaaggatat    1020
   gggctcactg agactacatc agctattctg attacacccg agggggatga taaaccgggc    1080
   gcggtcggta aagttgttcc attttttgaa gcgaaggttg tggatctgga taccgggaaa    1140
   acgctgggcg ttaatcaaag aggcgaactg tgtgtgagag gtcctatgat tatgtccggt    1200
   tatgtaaaca atccggaagc gaccaacgcc ttgattgaca aggatggatg gctacattct    1260
   ggagacatag cttactggga cgaagacgaa cacttcttca tcgttgaccg cctgaagtct    1320
   ctgattaagt acaaaggcta tcaggtggct cccgctgaat tggaatccat cttgctccaa    1380
   caccccaaca tcttcgacgc aggtgtcgca ggtcttcccg acgatgacgc cggtgaactt    1440
   cccgccgccg ttgttgtttt ggagcacgga aagacgatga cggaaaaaga gatcgtggat    1500
   tacgtcgcca gtcaagtaac aaccgcgaaa aagttgcgcg gaggagttgt gtttgtggac    1560
   gaagtaccga aaggtcttac cggaaaactc gacgcaagaa aaatcagaga gatcctcata    1620
   aaggccaaga agggcggaaa gatcgccgtg taa                                 1653
<210> 12
   <211> 940
   <212> DNA
   <213> Rattus norvegicus
<400> 12
   atgacttcga aagtttatga tccagaacaa aggaaacgga tgataactgg tccgcagtgg      60
   tgggccagat gtaaacaaat gaatgttctt gattcattta ttaattatta tgattcagaa     120
   aaacatgcag aaaatgctgt tattttttta catggtaacg cggcctcttc ttatttatgg     180
   cgacatgttg tgccacatat tgagccagta gcgcggtgta ttataccaga ccttattggt     240
   atgggcaaat caggcaaatc tggtaatggt tcttataggt tacttgatca ttacaaatat     300
   cttactgcat ggtttgaact tcttaattta ccaaagaaga tcatttttgt cggccatgat      360
   tggggtgctt gtttggcatt tcattatagc tatgagcatc aagataagat caaagcaata     420
   gttcacgctg aaagtgtagt agatgtgatt gaatcatggg atgaatggcc tgatattgaa     480
   gaagatattg cgttgatcaa atctgaagaa ggagaaaaaa tggttttgga gaataacttc     540
   ttcgtggaaa ccatgttgcc atcaaaaatc atgagaaagt tagaaccaga agaatttgca     600
   gcatatcttg aaccattcaa agagaaaggt gaagttcgtc gtccaacatt atcatggcct     660
   cgtgaaatcc cgttagtaaa aggtggtaaa cctgacgttg tacaaattgt taggaattat     720
   aatgcttatc tacgtgcaag tgatgattta ccaaaaatgt ttattgaatc ggacccagga     780
   ttcttttcca atgctattgt tgaaggtgcc aagaagtttc ctaatactga atttgtcaaa     840
   gtaaaaggtc ttcatttttc gcaagaagat gcacctgatg aaatgggaaa atatatcaaa     900
   tcgttcgttg agcgagttct caaaaatgaa caacgggccc                           940
<210> 13
   <211> 320
   <212> DNA
   <213> Rattus norvegicus
<400> 13
   tggatttatg ttgtatagat tatattgaga caaaaattat ctatttgtat atatacataa      60
   cagggtaaat tattcagtta agaaaaagat aattttatga actgcatgta taaatgaagt     120
   ttatacagta cagtggttct acaatctatt tattggacat atccatgacc agaaagaaac     180
   agtcatttgc gcacaacttt aaaagtctgc attacattcc tcgataatgt tgtggtttgt     240
   tgccgttgcc aagaattgaa aacaaaaagt taaaaaaaat aataaattgc atgctgcttt     300
   aattgtgaat tgataataaa                                                 320
<210> 14
   <211> 2791
   <212> DNA
   <213> Rattus norvegicus
<400> 14
   tggatttatg ttgtatagat tatattgaga caaaaattat ctatttgtat atatacataa      60
   cagggtaaat tattcagtta agaaaaagat aattttatga actgcatgta taaatgaagt     120
   ttatacagta cagtggttct acaatctatt tattggacat atccatgacc agaaagaaac     180
   agtcatttgc gcacaacttt aaaagtctgc attacattcc tcgataatgt tgtggtttgt     240
   tgccgttgcc aagaattgaa aacaaaaagt taaaaaaaat aataaattgc atgctgcttt     300
   aattgtgaat tgataataaa ctgtccctct ttcagaaaac agacaaaaaa acaaaaaaca     360
   aaaaaaagca aaaacaaaaa tttgaaccaa aacattccgt ttacatttta gacactaagt     420
   atcttcgttc ttgttagtac tctgttctac tgctttcaac ttctcatagc gttggaatta     480
   aaactatgtc aaggtgctgt tgtcattgct ttactggctt aggggatggg gaacgggagg     540
   ggtagatttc tgtttgtttt gtgttttatt tcgtttgttt gtttgttttg tttttttagt     600
   tccacccgga gtagggatgg agaaaatttc ttcactctcc attctggttg ataaagcgtt     660
   acatttgtat gttgtaaaaa atgtttgcaa aatccaatca gatgactgga aaacgaataa     720
   aaattaaggc aactgaataa aatgctcaca caacactgcc catgatgtat ctccctggtc     780
   ccccaggtca ctcttctggc atgggtcagg gaaagctgct tttattggaa agaccagcat     840
   ttgtttaaag cacattcttt ccctccctcc tcccattttg gtccctttct tttttgtttt     900
   gttttaagaa agaaaattaa gttgcgcgct ttgaaatatt ttatcactgc tgtgaacaga     960
   tgaacaatgt gtgtcatttc atgacactcg tggaaaacag tgattttttt tttatttttt    1020
   gccctaagga gaaacaagta agaataaccg aaaatgttct ttttttttaa aggcataaac    1080
   agtggataag ttataatatg gcctaacaat gtttgcagat aaaagatatt gcatacagcc    1140
   agatactaga gcagggatcc acactgccac tgaaatgcga ctgaatggcc ctgtggaggc    1200
   taagtggagc tgacatacta tttcctggca gagcaggagg aatttctgag tggccatcct    1260
   gaggtctaga tggaggtggg gaatggtact tgagacattc ctaaaggaag gctcggaagc    1320
   acccttcaga gcaggctctg gaatgatgtg tcaagtttct taggccttct gctttaagtg    1380
   cctacgttac ctaacagtgc tcaagaggtt ctcaattgga gaaccacact caaatccatt    1440
   tatggcctcc atcccatttt aaataattat ggataaagtt ggattaacct ggagcagctt    1500
   tggatccaaa tatggcatag cagtgatgct atcagtgcag catgatggga aatgtttgct    1560
   gtgaagagac ttaactttct ttgcgcttag acttcaggaa gcctaggttt tatttattta     1620
   tttttttgag acattttggt gaaaggaaaa aagaaagaag aaaacaaaca aacaaaacca     1680
   gaaaaagcac caaaacttag gcagaatgag caatgtctgt ctgtaagggc tagaatgaca     1740
   aggcatagga aggtgctttc actgtgaaag agacaagaac acaggaggaa atactgctta     1800
   agtgaagagc acagaaagct cctgatagtt ctgtccattc agcacaaggg tcccttctac     1860
   actttacctc ttggggttag gagaagtcaa gctggaagcc tgaatgaatg gccccaatga     1920
   gaactagtgt taagcccatt tccctagtga ggttttccgc cagcgcgaat gtgttagtgg     1980
   ttacctgact gggctcctgg gcatcagaaa aagaggcaaa caattgcttc atcttaggag     2040
   tggaaagggt gaaacaaagt ggctgtccac tgtgactcag ggagtgaaga taccatcagc     2100
   aaatagtttc tttttgttca actgttcctt tagaactagt ctgtcttctg gagtcccact     2160
   gaatcccctg tttttggaag acttcacgta gcctagattg ttttgtgccg tttgacaaca     2220
   ttaatctctg tcatcatttt taacctatta aggaatgctt tgaatatctg ctatatgcta     2280
   actttttgca gcttcattct gagagacgtt agtcaaacaa ataaaaggag ccccatcaca     2340
   atctcacggt attcgaaggg ccaggtcgat taggtggctt cataggagac cctccgcaac     2400
   tgtgtggtca gtggctggct ctcataccca ctaagataca tcatagctcc atgtcggtgg     2460
   tttatgttga cctgagattg atttgttaaa atctctcctc tgtttctgtt cgttctgttt     2520
   ccgtcctgtt ctgttctgtt ctgttctgaa agtcttgctg tggtctcttt ttggcagaag     2580
   tgtttcatgc atggcagcag gcctgatgct ttttatagtg attcccattg aaactgtaag     2640
   taaatgtctg tggccttgtt ctctctatgg taaagatatt attcaccatg taaaacaaga     2700
   aaaatattta ttgtatttta gtatatttat ataattatgt tattgaaaaa aattggcatt     2760
   aaaacttaac cacatcagaa gcctattgta a                                    2791

## Description

### FIELD OF THE INVENTION

The present invention concerns a method for the identification of specific compounds able to modulate Brain-derived neurotrophic factor (BDNF), which can be used as drugs for the treatment of neurological and neuropsychiatric diseases and which are suitable for the treatment of deleterious effects caused to the nervous system by abuse of illegal or legal drugs.

### STATE OF THE ART

Brain-derived neurotrophic factor (BDNF) is a growth and trophic factor that belongs to the family of related secretory proteins called neurotrophins. BDNF plays multifaceted and in part opposed functions in both development and maintenance of the nervous system. Indeed, BDNF promotes both cell survival and cell death, neuronal maturation including neurites and dendritic spine outgrowth, and has a prominent role in various forms of synaptic plasticity such as long-term potentiation and long-term depression (Lu, Hidaka et al. 1996; Huang and Reichardt 2001).

Transcription of the BDNF gene produces 11 primary transcripts in rodents (Aid, Kazantseva et al. 2007; Gene ID: 12064 and 24225) and 17 primary transcripts in humans (Pruunsild, Kazantseva et al. 2007; Gene ID: 627) each characterized by a different 5' untranslated (UTR) exon linked by alternative splicing to a common 3' exon coding for the protein and 3'UTR. Since the 3'UTR contains two polyadenylation sites, each primary transcript can exist in two forms, one with a short and the other with a long 3' UTR, producing a total of 22 (in rodents) or 34 (in humans) possible transcripts.

Lau A.G. et al. (Proc Natl Acad Sci USA. 2010 Sep 7;107(36):15945-50) reports the differential regulation of BDNF translation in brain neurons, and in particular the finding that the long BDNF 3'UTR is a bona fide cis-acting translation suppressor at rest whereas the short 3'UTR mediates active translation to maintain basal levels of BDNF protein production.

Intraperitoneal injection of pro-convulsant agents such as kainic acid or pilocarpine in adult rats, induces a segregation of exonl and 4 BDNF transcripts in the cell soma of hippocampal and cortical neurons, whereas exons 2B, 2C, and 6 transcripts display a somatodendritic distribution extended into distal dendrites (Pattabiraman, Tropea et al. 2005; Chiaruttini, Sonego et al. 2008). This differential local expression of BDNF transcripts appears to be a general mechanism used throughout the nervous system as it is found in cortex, hippocampus and hypothalamus (Pattabiraman, Tropea et al. 2005; Chiaruttini, Sonego et al. 2008; Aliaga, Mendoza et al. 2009). Of note, BDNF protein generated from each individual transcript segregates to the same subcellular domain where the transcript that has generated it localizes, regulating BDNF availability in specific subcellular regions of the neurons (Tongiorgi and Baj 2008). On this basis, it has been proposed that BDNF transcripts may represent a spatial code in which the different variants are used for the delivery of BDNF mRNA and accumulation of locally produced protein at specific subsets of synapses ((Chiaruttini, Sonego et al. 2008; Tongiorgi and Baj 2008).

Since pyramidal neurons of cortical layer 5 in the visual cortex as well as hippocampal neurons, receive segregated synaptic inputs from GABAergic interneurons on the cell soma and glutamatergic inputs on dendrites, the different BDNF transcripts may be recruited to modulate specifically different types of synaptic contacts (Tongiorgi, 2008). Accordingly, mice with a selective ablation of the "somatic" exon 4 transcript were shown to exhibit significant deficits in GABAergic interneurons in the prefrontal cortex, particularly those expressing parvalbumin, a subtype implicated in executive function and schizophrenia. Moreover, disruption of promoter 4-driven BDNF transcription impaired inhibitory but not excitatory synaptic transmission recorded from layer 5 pyramidal neurons in the prefrontal cortex (Sakata, Woo et al. 2009).

Numerous studies have reported changes in the expression of the different BDNF mRNA variants in various brain diseases such as epilepsy (Aid, Kazantseva et al. 2007) and mental retardation Rett Syndrome (Chen, Chang et al. 2003), or neurodegenerative diseases like Huntington disease (Zuccato, Liber et al. 2005) and Alzheimer's disease (Garzon, Yu et al. 2002). Moreover, BDNF variants levels are different from normal brains in various human brain regions from cocaine addicts. The cocaine group showed threefold higher levels of exon 4-specific mRNAs in cerebellum versus controls and a 40% reduction of exon 4 and exon 1-specific BDNF mRNA in the cortex (Jiang, Zhou et al. 2009). Fear and stress can also specifically affect BDNF transcripts expression levels. A recent study reported evidence of an epigenetic regulation of BDNF gene transcription in the consolidation of fear memory. Contextual fear learning induced differential regulation of exon-specific BDNF mRNAs (1, 4, 6, 9a) that was associated with changes in BDNF DNA methylation and altered local chromatin structure (Roth, Lubin et al. 2009).

Bandyopadhyay S. et al. (J Biomol Screen. 2006 Aug;11 (5):469-80) describes a stable cell line expressing luciferase under the control of the 5'UTR of the amyloid precursor protein, but does not mention the 3'UTR.

WO2004065561 describes methods for screening and identifying compounds that modulate the VEGF gene by means of a nucleic acid comprising a reporter gene operably linked to a UTR of the same VEGF gene. Vectors are constructed with the one, both or none of the 5' and 3' UTRs, and results show that a 3-fold increase in firefly luciferase expression can be detected only in the expression vectors that contain the 5'UTRs.

Pruunsild et al. (2007), and Aid et al. (2007) studies have highlighted that the 5'UTR region of each BDNF transcript is encoded by a different exon with unique length, GC content and putative secondary structures. Thus, each BDNF transcript is likely to display a different translatability. However, since the final protein product is the same, it is presently impossible to determine the relative contribution of each single BDNF splice variants to the production of the BDNF protein. Thus, to determine the role of each BDNF transcript in producing the protein, it is necessary to develop new tools able to determine the amount of protein generated by each BDNF splice variant at basal condition and in response to a disease state or a specific drug. Clearly, a change in mRNA levels of transcript that is poorly translatable will have less impact on total BDNF levels than a highly translatable splice variant.

The need and importance is increasingly felt for the identification of specific compounds able to modulate BDNF, which can be used as drugs for the treatment of neurological and neuropsychiatric diseases or for the treatment of deleterious effects caused to the nervous system by abuse of illegal or legal drugs. It is therefore object of the present invention the development of a method of screening for BDNF translation modulators which allow the measurement of BDNF protein production. In particular it would be highly desirable to identify a screening method which would allow to determine the expression of all possible BDNF variants, and allow, at the same time, to obtain information on the final amounts of the BDNF protein produced in response to a specific drug.

### SUMMARY OF THE INVENTION

Object of this invention is the creation of a cell-based screening assay to screen for natural or synthetic compounds able to increase or decrease the neurotrophin brain-derived neurotrophic factor (BDNF) protein levels produced by translation of the different BDNF mRNA variants.

In particular, the present invention concerns a method of screening for modulators of the translation of brain-derived neurotrophic factor (BDNF) comprising the steps of:
a) transfecting a mammalian cell with a nucleic acid construct, said nucleic acid construct comprising:
   - a 5' untranslated (5'UTR) exon of a mammalian BDNF gene and a 3' untranslated (3'UTR) exon of a mammalian BDNF directly linked to the cDNA of a functional reporter gene, in the absence of any other BDNF promoter sequence;
b) contacting said mammalian cell with a screening compound;
c) detecting the luminescence produced by said mammalian cell of step b);
d) comparing the luminescence measurements of the cell contacted with the screening compound with the luminescence measurements of a control;
wherein a change in luminescence compared to the control allows to identify a modulator of BDNF translation.

The present invention further concerns a nucleic acid construct comprising:
a 5' untranslated (5'UTR) exon of the rat BDNF gene selected from the group consisting of SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9 or SEQ ID NO. 10 directly linked to the cDNA of a functional reporter gene, in the absence of any other BDNF promoter sequence; and
a 3' untranslated (3'UTR) exon of the rat BDNF gene selected from the group consisting of SEQ ID NO. 13 and SEQ ID NO. 14.

A still further object of the present invention is a plasmid comprising the nucleic acid construct according to the present invention.

### BRIEF DESCRIPTION OF THE DRAWINGS

The characteristics and advantages of the present invention will be apparent from the detailed description reported below, from the Examples given for illustrative and non-limiting purposes, and from the annexed Figures 1-6, wherein:
Figure 1: shows the strategy of cloning for creation of the new pFluc-N1 and pRIuc vectors described in Example 1.
Figure 2A: shows the mRNA levels for each transcript transfected in SH-SY5Y and PCR-amplified with primers specific for the luciferase reporter gene as described in Example 2;
Figure 2B: shows the densitometric analysis normalized for the levels of the housekeeping gene GAPDH revealed some variability in mRNA expression for the different variants as described in Example 2;
Figure 2C: shows the results of normalization of the luciferase translation data for the levels of the corresponding mRNA lead to the results as described in Example 3;
Figure 2D shows the results of a western-blot, showing the protein expression (antibody anti-luc) for each pN1-insert name as described in Example 3.
Figure 3: shows the effects of drug treatment on the translation of BDNF 5'UTRs as described in Example 4.
Figure 4: shows MTT viability assays demonstrating that none of the drugs used at the indicated concentrations causes death in SY-SY5Y human neuroblastoma cells as described in Example 4.
Figure 5: shows the effects of drug treatment on the translation of BDNF 3'UTRs as described in Example 5.
Figure 6: shows the inhibition of KCI-induced translation of the ex6-Fluc-3'UTRIong construct as described in Example 6.

### DETAILED DESCRIPTION OF THE INVENTION

The present invention concerns a method of screening for modulators of the translation of brain-derived neurotrophic factor (BDNF) comprising the steps of:
a) transfecting a mammalian cell with a nucleic acid construct, said nucleic acid construct comprising:
   - a 5' untranslated (5'UTR) exon of a mammalian BDNF gene and a 3' untranslated (3'UTR) exon of a mammalian BDNF directly linked to the cDNA of a functional reporter gene, in the absence of any other BDNF promoter sequence;
b) contacting said mammalian cell with a screening compound;
c) detecting the luminescence produced by said mammalian cell of step b);
d) comparing the luminescence measurements of the cell contacted with the screening compound with the luminescence measurements of a control;
wherein a change in luminescence compared to the control allows to identify a modulator of BDNF translation.

In a preferred aspect, the present invention regards a method of screening for modulators of the translation of brain-derived neurotrophic factor (BDNF), wherein said 5' untranslated (5'UTR) exon of a mammalian BDNF gene is the 5' untranslated (5'UTR) exon of the rat BDNF gene.

In a more preferred embodiment, the present invention regards a method of screening for modulators of the translation of brain-derived neurotrophic factor (BDNF), wherein said 5' untranslated (5'UTR) exon of the rat BDNF gene is selected from the group consisting of SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9 or SEQ ID NO. 10.

For the purposes of the present invention, each 5' untranslated (5'UTR) exon of the rat BDNF gene has a corresponding SEQ ID NO. as follows:
SEQ ID NO. 1 corresponds to the nucleotidic sequence of exon 1 of the 5' untranslated (5'UTR) exon of the rat BDNF gene;
SEQ ID NO. 2 corresponds to the nucleotidic sequence of exon 2a of the 5' untranslated (5'UTR) exon of the rat BDNF gene;
SEQ ID NO. 3 corresponds to the nucleotidic sequence of exon 2b of the 5' untranslated (5'UTR) exon of the rat BDNF gene;
SEQ ID NO. 4 corresponds to the nucleotidic sequence of exon 2c of the 5' untranslated (5'UTR) exon of the rat BDNF gene;
SEQ ID NO. 5 corresponds to the nucleotidic sequence of exon 3 of the 5' untranslated (5'UTR) exon of the rat BDNF gene;
SEQ ID NO. 6 corresponds to the nucleotidic sequence of exon 4 of the 5' untranslated (5'UTR) exon of the rat BDNF gene;
SEQ ID NO. 7 corresponds to the nucleotidic sequence of exon 5 of the 5' untranslated (5'UTR) exon of the rat BDNF gene;
SEQ ID NO. 8 corresponds to the nucleotidic sequence of exon 6 of the 5' untranslated (5'UTR) exon of the rat BDNF gene;
SEQ ID NO. 9 corresponds to the nucleotidic sequence of exon 7 of the 5' untranslated (5'UTR) exon of the rat BDNF gene;
SEQ ID NO. 10 corresponds to the nucleotidic sequence of exon 8 of the 5' untranslated (5'UTR) exon of the rat BDNF gene.

For the purposes of the present invention, by the term "BDNF", it is intended any gene of a mammalian brain-derived neurotrophic factor which can generate any isoform of the BDNF protein such as proBDNF, truncated or mature BDNF, in particular a rat BDNF. In addition to rat BDNF, BDNF sequences for human and other mammalian species are known in the art and sequences of BDNF from these other species can be found on the NCBI website and in other resources known to persons of skill in the art.

According to a preferred aspect, the present invention regards a method of screening for modulators of the translation of brain-derived neurotrophic factor (BDNF), wherein said 3' untranslated (3'UTR) exon of a mammalian BDNF is the 3' untranslated (3'UTR) exon of rat BDNF gene.

In a more preferred embodiment, the present invention regards a method of screening for modulators of the translation of brain-derived neurotrophic factor (BDNF), wherein said 3' untranslated (3'UTR) exon of the rat BDNF gene is selected from the group consisting of SEQ ID NO. 13 and SEQ ID NO. 14.

For the purposes of the present invention, each 3' untranslated (3'UTR) exon of the rat BDNF gene has a corresponding SEQ ID NO. as follows:
SEQ ID NO. 13 corresponds to the nucleotidic sequence of the short 3' untranslated region of the rat BDNF gene.
SEQ ID NO. 14 corresponds to the nucleotidic sequence of the long 3' untranslated region of the rat BDNF gene.

In the present invention by "screening compound", a compound that could be a promising candidate for drug development is intended. Such chemical compounds interact with a target protein and are therefore potential candidates for drug development. The target protein of the present invention is a protein involved in the signalling cascades which regulate the translation of a transcript encoding BDNF and of which non-limiting examples are represented by: 1) ion channels; 2) protein involved in neurotransmitters, peptides, hormons, and trophic factors release, synthesis or re-uptake; 3) receptors for neurotransmitters, peptides, hormons and trophic factors; 4) enzymes involved in post-translational modifications of proteins of which non-limiting examples are represented by phosphorylation, acetylation, or sumoylation; 5) enzymes involved in production of small signalling molecules of which non-limiting examples are represented by nitric-oxide, cyclic nucleotides, phospholipids; 6) translation factors or proteins regulating the activity of translation factors, and 7) RNA-binding proteins or proteins regulating the activity of RNA-binding proteins involved in RNA synthesis, trafficking, folding, translation or degradation.

The method of the present invention has the advantage of being useful for the identification of specific compounds able to modulate BDNF, which can be used as drugs for the treatment of neurological and neuropsychiatric diseases or for the treatment of deleterious effects caused to the nervous system by abuse of illegal or legal drugs.

A further advantage of the method of the present invention is that of allowing the screening for BDNF translation modulators which allow the measurement of BDNF protein production, and in particular allows to determine the expression of all possible BDNF variants, and allow, at the same time, to obtain information on the final amounts of the BDNF protein produced in response to a specific drug.

A further object of the present invention is a method of screening for modulators of brain-derived neurotrophic factor (BDNF), wherein said mammalian cell grows in adhesion.

In the present invention, said mammalian cell is chosen from the group consisting of human neuroblastoma-derived cell lines of which non-limiting examples are represented by SH-SY5Y, SK-N-SH, and SK-N-BE cells, or glioblastoma-derived cell lines of which non- limiting examples are represented by the GBM cell lines U87, U343, U563 and the MB cell lines D283, DAOY, and SNB40 or immortalized human cell lines of neuronal origin of which non-limiting examples are represented by human neural progenitor cells (hNPCs), human embryonic spinal cord-derived cell line (HSP1), human dorsal root ganglion cell line, human cortical neural progenitor cells, or of human cell lines of non-neuronal origin of which non-limiting examples are represented by NIH-3T3 cells, HeLa cells, or rodent immortalized cell lines of which non-limiting examples are represented by HEK 293 cells, and CHO cells.

In a preferred embodiment, the present invention concerns a method of screening for modulators of BDNF, wherein said mammalian cell is chosen from the group consisting of SH-SY5Y cells, SK-N-SH cells, SK-N-BE cells, NIH-3T3 cells, HeLa cells, HEK 293 cells, and CHO cells.

The present invention further relates to a method of screening for modulators of BDNF, wherein said screening compound is a BDNF-translation agonist.

The present invention further relates to a method of screening for modulators of BDNF, wherein said screening compound is selected from the group consisting of ion-channels agonists, neurotransmitters synthesis, release and re-uptake agonists, neurotransmitter receptors agonists, growth-factors receptors agonists, translation factors agonists and intracellular signalling agonists.

The present invention further relates to a method of screening for modulators of BDNF, wherein said screening compound is a BDNF-translation antagonist.

The present invention further relates to a method of screening for modulators of BDNF, wherein said screening compound is selected from the group consisting of ion-channels antagonists, neurotransmitters synthesis, release and re-uptake antagonists, neurotransmitter receptors antagonists, growth-factors receptors antagonists, translation factors antagonists and intracellular signalling antagonists.

Object of this invention is therefore the creation of a cell-based screening assay to screen for natural or synthetic compounds able to treat neurological diseases through an increase or decrease of BDNF protein levels produced by translation of the different BDNF splice variants.

In a further embodiment, the present invention regards a method of screening for modulators of BDNF, wherein said reporter gene is a luciferase gene.

In a further embodiment, the present invention regards a method of screening for modulators of BDNF, wherein said luciferase gene is chosen from the group consisting of Firefly luciferase and Renilla luciferase.

For the purposes of the present invention, each luciferase gene has a corresponding SEQ ID NO. as follows:
SEQ ID NO. 11 corresponds to the nucleotidic sequence of the luciferase gene of the firefly *Photinus pyralis* (full coding sequence)
SEQ ID NO. 12 corresponds to the nucleotidic sequence of the luciferase gene of the sea pansy *Renilla reniformis* (full coding sequence).

In a still further embodiment the present invention regards a method of screening for modulators of the translation of a brain-derived neurotrophic factor (BDNF), wherein said method of screening is carried out by the high throughput screening (HTS) technique.

In the present invention by the High-throughput screening (HTS) technique, the technique which seeks to identify potential candidates for drug development is intended. HTS is a technique for scientific experimentation especially used in drug discovery. Using robotics, data processing and control software, liquid handling devices, and sensitive detectors, HTS allows to quickly conduct millions of biochemical, genetic or pharmacological tests.

In a still further embodiment the present invention regards a nucleic acid construct comprising:
a 5' untranslated (5'UTR) exon of the rat BDNF gene selected from the group consisting of SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9 or SEQ ID NO. 10 directly linked to the cDNA of a functional reporter gene, in the absence of any other BDNF promoter sequence;
and a 3' untranslated (3'UTR) exon of the rat BDNF gene selected from the group consisting of SEQ ID NO. 13 and SEQ ID NO. 14.

In a still further embodiment the present invention regards a nucleic acid construct, wherein said reporter gene is a luciferase gene.

In a still further embodiment the present invention regards a nucleic acid construct, wherein said luciferase gene is chosen from the group consisting of Firefly luciferase and Renilla luciferase.

In a still further embodiment the present invention regards a plasmid comprising the nucleic acid construct according to the present invention.

### EXAMPLES

### Example 1.

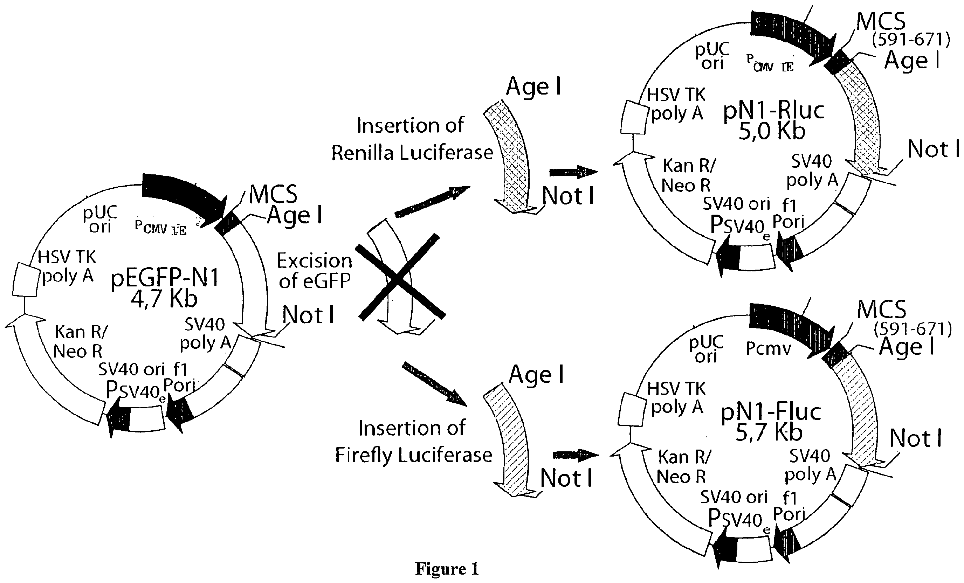

### Cloning and generation of Luciferase constructs pN1-RLuc and pN1-FLuc as shown in Figure 1

Two different constructs were generated by modifying the commercial pEGFP-N1 plasmid (Clontech). pN1-Rluc was obtained by the replacement of the EGFP coding sequence of pEGFP-N1, with the Renilla-luciferase (GI:2582516; GB:AAB82577.1). pN1-FLuc was obtained by the replacement of the EGFP coding sequence of pEGFP-N1, with the Firefly-luciferase open reading frame (ORF) (GI:13195704; GB:AAA89084.1).

The cloning strategy and the resulting maps of the newly obtained pN1-FLuc (pFluc) and pN1-RLuc (or pRluc) are shown in Figure 1. pN1-FLuc (pFluc) and pN1-RLuc (or pRIuc) were then used as a backbone to construct the vectors containing the different BDNF splicing variants.

### Cloning strategy

In detail, the pEGFP-N1 vector was modified so that the sequence between the restriction sites Age I and Not I, encoding the EGFP (enhanced green fluorescent protein) was removed. The digested backbone vector was purified through DNA agarose gel extraction (Sigma Aldrich, Gel Extraction kit). The Firefly luciferase DNA fragment (Rluc) was obtained by PCR amplification of the luc+ gene from the commercial vector pGL3 Basic and the Renilla luciferase DNA fragment (Rluc) was obtained by PCR amplification of the Rluc gene from the pRL-SV40 Vector (both purchased from Promega Corporation).

The Fluc and Rluc ORFs were amplified using Phusion high-fidelity DNA Polymerase (Finnzymes) 0,02 U/µl. Primers were purchased from Eurofins MWG GmbH. The PCR conditions and primers sequence are summarized in the Table 1.

**Table 1. PCR primers and conditions for amplifying Rluc and Fluc**

| | **Primer Forward** | **Primer Reverse** | **PCR CONDITIONS** | | | |
|---|---|---|---|---|---|---|
| Fluc | | | Denaturation | 98°C | 10 sec | 30 cycles |
| | | | Annealing | 58°C | 20sec | |
| | | | Extension | 72°C | 40 sec | |
| Rluc | | | Denaturation | 98°C | 10 sec | 30 cycles |
| | | | Annealing | 57,5°C | 20sec | |
| | | | Extension | 72°C | 30 sec | |

PCR-amplified Fluc and Rluc fragments were cut with the restriction enzymes Agel and Notl (New England Biolabs) and then ligated into the pEGFP-N1 vector backbone without EGFP (as described above).

In order to obtain one specific expression vector for each BDNF variant (from now on, named pN1-insert name-Fluc), the sequence of single rat BDNF exons 1-8 encoding the alternatively spliced 5'UTR regions, was amplified by PCR starting from the cDNA obtained from total RNA extracted from adult rat brain and retro-transcribed using Superscript-II transcriptase (Promega). The forward primers, specific for each 5'exon contained the Xhol restriction site and the common reverse primer in the coding region contained the Agel restriction site (Table 2). PCR amplicons were cloned between the Xhol and Agel restriction sites of the pFluc-N1 vector upstream of the Firefly Luciferase coding sequence. In addition, the complete rat BDNF coding sequence (CDS) was cloned between the Xhol and Sacll restriction site of the pFluc-N1 vector upstream the Fluc gene.

Furthermore, the two 3'untranslated regions (3'UTR) variants, called respectively 3'UTR-long and 3'UTR-short were cloned into the HpaI and NotI restriction sites of pN-Fluc vector downstream the F-luc gene using specific primers generating specific vectors (from now on named, pN1-Fluc-3'UTR-long or short; or pN1-insert name-Fluc-3'UTR-long or short. See Table I). Finally, the two Kozak sequences, one specific for exon 1 and the other in common to all other transcripts (exon 9), were inserted (Agel-Notl) through a modified Fluc sequence containing the two above modified kozak sequences. The BDNF CDS, 3'UTR-long and 3'UTR-short and all exons were amplified using Phusion high-fidelity DNA Poliymerase (Finnzymes) 0,02 U/µl (or Platinum Polymerase for exon 3). Primers were purchased from Eurofins MWG GmbH (see Table 2 for PCR conditions).

### Results

The plasmids generated with the cloning strategy described above are listed in Table 3.

**Table 2. PCR primers and conditions for pN1-BDNF insert -Fluc vectors**

| | **Primer Forward** | **Primer Reverse** | **PCR CONDITIONS** | | | |
|---|---|---|---|---|---|---|
| 3'UTR long | | | Denaturation | 98°C | 10 sec | 31 cydes |
| | | | Annealing | 57,5°C | 15sec | |
| | | | Extension | 72°C | 2 min | |
| 3' UTR short | | | Denaturation | 98°C | 10 sec | 31 cydes |
| | | | Annealing | 57,5°C | 15 sec | |
| | | | Extension | 72°C | 15 sec | |
| CDS | | | Denaturation | 98°C | 10 sec | 31 cydes |
| | | | Annealing | 57,5°C | 15 sec | |
| | | | Extension | 72°C | 40 sec | |
| Exon 1 | | | Denaturation | 98°C | 10 sec | 31 cydes |
| | | | Annealing | 57°C | 20 sec | |
| | | | Extension | 72°C | 40 sec | |
| Exon 2A | | | Denaturation | 98°C | 10 sec | 31 cydes |
| | | | Annealing | 58°C | 20 sec | |
| | | | Extension | 72°C | 35 sec | |
| Exon 2B | | | Denaturation | 98°C | 10 sec | 31 cydes |
| | | | Annealing | 58°C | 20 sec | |
| | | | Extension | 72°C | 35 sec | |
| Exon 2C | | | Denaturation | 98°C | 10 sec | 31 cydes |
| | | | Annealing | 58°C | 20 sec | |
| | | | Extension | 72°C | 35 sec | |
| Exon 3* | | | Denaturation | 94°C | 15 sec | 31 cydes |
| | | | Annealing | 55°C | 15 sec | |
| | | | Extension | 68°C | 20 sec | |
| Exon 4 | | | Denaturation | 98°C | 10 sec | 31 cydes |
| | | | Annealing | 58°C | 20 sec | |
| | | | Extension | 72°C | 20 sec | |
| Exon 5 | | | Denaturation | 98°C | 10 sec | 31 cydes |
| | | | Annealing | 58°C | 20 sec | |
| | | | Extension | 72°C | 15 sec | |
| Exon 6** | | | Denaturation | 98°C | 10 sec | 31 cydes |
| | | | Annealing | 57,5°C | 20 sec | |
| | | | Extension | 72°C | 15 sec | |
| Exon 7 | | | Denaturation | 98°C | 10 sec | 31 cydes |
| | | | Annealing | 58°C | 20 sec | |
| | | | Extension | 72°C | 20 sec | |
| Exon 8 | | | Denaturation | 98°C | 10 sec | 31 cydes |
| | | | Annealing | 58°C | 20 sec | |
| | | | Extension | 72°C | 35 sec | |

| **Complete list of the plasmid\|s** | | | | |
|---|---|---|---|---|
| | | | | |
| 1) | pN1-Rluc | | 12) | pN1-exon4-Fluc |
| 2) | pN1-Fluc | | 13) | pN1-exon7-Fluc |
| 3) | pN1-Fluc-3'short | | 14) | pN1-exon5-Fluc |
| 4) | pN1-Fluc-3'long | | 15) | pN1-exon3-Fluc |
| 5) | pN1-CDS-Fluc | | 16) | pN1-exon6-Fluc |
| 6) | pN1-kozakex9-Fluc | | 17) | pN1-exon1-Fluc-3'UTR short |
| 7) | pN1-exon2B-Fluc | | 18) | pN1-exon1-Fluc-3'UTR long |
| 8) | pN1-exon2A-Fluc | | 19) | pN1-exon4-Fluc-3'UTR short |
| 9) | pN1-exon8-Fluc | | 20) | pN1-exon4-Fluc-3'UTR long |
| 10) | pN1-exon2C-Fluc | | 21) | pN1-exon6-Fluc-3'UTR short |
| 11) | pN1-exon1-Fluc | | 22) | pN1-exon6-Fluc-3'UTR long |

medium was replaced with fresh medium after 24h, before transfection.

### Transfection

Plasmids were transfected into SH-SY5Y cells using lipofectamine 2000 (Invitrogen) to obtain a transient transfection. The relative quantity of DNA to lipofectamine has been determined and the optimal ratio was found at 0,2 µg of DNA and 0,5 µl of lipofectamine for each well of a 96 multiwell plate. At the time of transfection SH-SY5Y cells were around 70-75% of confluency. According to the Promega protocol for luciferase activity measurements, each construct-containing Fluc was transfected together with the normalizing Rluc construct with a 10:1 ratio (0,2 µg Fluc containing vector together with 0,02 µg of pN1-Rluc vector). The DNA (0,2 µg) was mixed with 25 µl of native MEM medium and incubated for 5 minutes at room temperature. The two solutions were then mixed and incubated for 20 minutes at room temperature to allow the formation of DNA-containing micelles. Then, 50 µl of such solution were added in each well to cells and incubated for 24h at 37°C. After 24h transfection, the medium was removed and replaced with fresh culture medium.

### Results

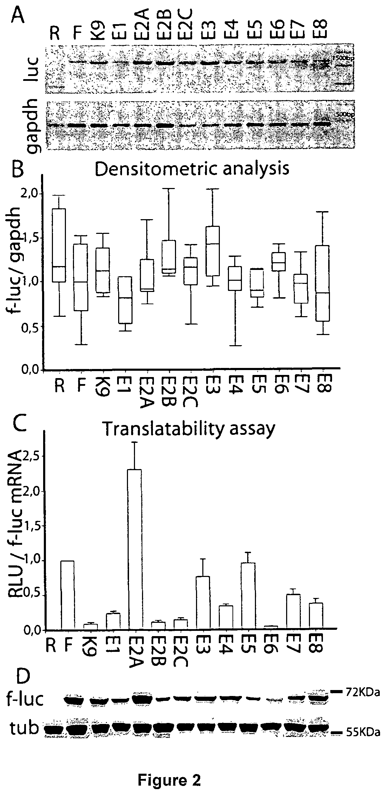

Figure 2A shows the mRNA levels for each transcript transfected in SH-SY5Y and PCR-amplified with primers specific for the luciferase reporter gene. In this way each transcript is amplified with the same efficiency. Densitometric analysis (Figure 2B) of three independent experiments (each in duplicate), normalized for the levels of the housekeeping gene GAPDH revealed some variability in mRNA expression for the different variants which was non-statistically significant.

### EXAMPLE 3

### Dual luciferase assay

Each vector was tested in the human neuroblastoma cell line SH-SY5Y and the basal levels of translation measured as F/R-luc ratio, were determined in three independent experiments (each in duplicate) in 96 well white plates with clear bottom (PBI International). To determine the effects of receptors activation on translation controlled by the different BDNF 5'UTR sequences, cells were treated for 3 hours with 50mM KCI to induce generalized cell depolarization.

### Firefly luciferase assay

Luciferase assay was performed according to the manufacturer's instructions (Promega). At the time of luciferase assay, the growth medium was removed from the cultured cells and a sufficient volume of PBS 1X was applied to wash the surface of the culture vessel. Then, the PBS 1X solution was completely removed and the cells lysis was started by adding 20 µl of PLB (passive lysis buffer) into each well. After adding the PLB solution, the culture plate was placed on an orbital shaker with gentle shaking at room temperature for 15 minutes to cover uniformly the cell monolayer with PLB. After lysis, 100 µl of Luciferase Assay Reagent II (LARII) were dispensed in each well and Firefly luciferase activity was measured using the Glo Max Multi luminometer with two injectors (Promega Corporation).

### Renilla luciferase assay

After quantifying the Firefly luminescence, this reaction was quenched and simultaneously, the Renilla luciferase reaction was initiated by adding 100 µl Stop & Glo Reagent (Promega) to the same well. Then, Renilla luciferase activity was measured using the Glo Max Multi luminometer with two injectors (Promega Corporation).

### Western-blot analysis

Adherent SH-SY5Y cells were mechanically removed with a cell scraper (Sarstedt) with 150µl of cold (4°C) lysis buffer containing 137mM NaCl (Fluka), 20mM Tris-HCl pH 8.0, 1% Nonidet P-40, 10% glycerol, and a cocktail of protease inhibitors - 1 mM PMSF (Phenilmethyl-sulfonilfluoride), 10µg/ml TEWI (Turkey Egg White inhibitor), 4µg/ml SBTI (Soy Bean Trypsin inhibitor), 1mM IAA (Iodoacetamide), 1mM SPERM (Spermidin). SH- SY5Y extracts were further homogenized with a syringe, rocked for 30-45min at 4ºC, and centrifuged at 10,000xg for 8-10min at 10ºC, to remove cellular debris. Protein homogenates (30µg) were separated on 12% SDS-polyacrylamide gels and transferred onto nitrocellulose Protran membranes (Whatman). After blocking (4% non-fat milk powder, 0,05% tween-20 in phosphate-buffered saline) at RT, membranes were divided into two halves at the level of the 60KDa marker and incubated overnight at 4°C with either a goat anti-firefly luciferase antibodies (AbCam; diluted 1:1000) or mouse monoclonal anti-tubulin antibody (YOL1/34, AbCam; diluted 1:2000). Following incubation with anti-goat or anti-mouse secondary antibodies conjugated with HRP (both DakoCytomation, diluted 1:10,000), immunoreactivity was detected by chemiluminescence (Amersham Biosciences). Films were scanned using an Epson Scanner (Epson perfection V500-photo).

### Results

The different 5'UTR BDNF sequences have a different translatability. Normalization of the luciferase translation data for the levels of the corresponding mRNA lead to the results shown in Figure 2C. Insertion of the different rat BDNF 5'UTR upstream to the luciferase gene resulted in a marked repression of translation of the reporter for most transcripts with the exception of exon 2A which enhanced translation. In Figure 2D a representative Western-blot is displayed, showing the protein expression (antibody anti-luc) for each pN1-insert name-Fluc construct. Results of the luciferase assay and the western-blot are consistent with each other.

### EXAMPLE 4

### Response of BDNF 5'UTR splice variants to different agonists

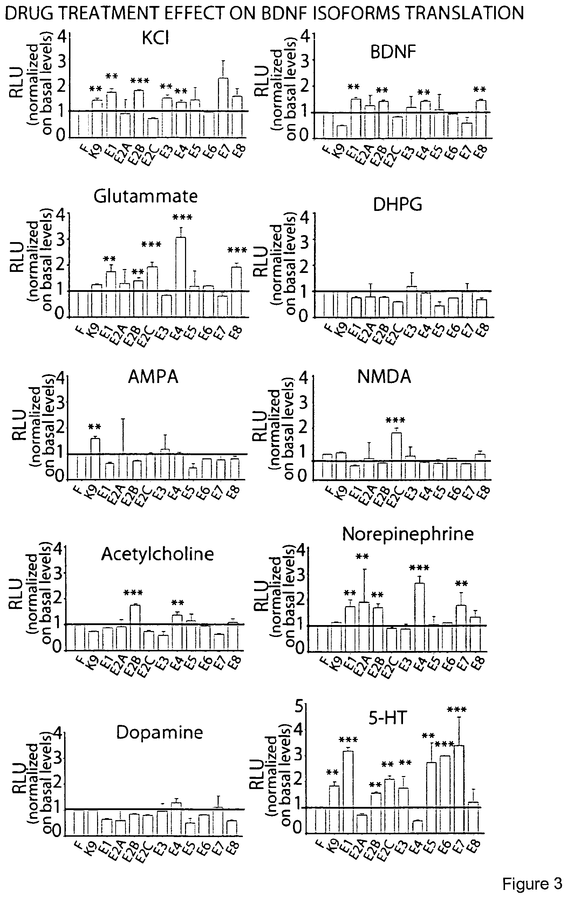

The different BDNF splice variants were analyzed for their response to different receptor agonists (Figure 3) in undifferentiated neuro-blastoma SH-SY5Y cells. One day prior transfection, undifferentiated neuro-blastoma SH-SY5Y cells were seeded into a 96-well white plate with clear bottom (PBI International) at a confluency of 50-60% and cultured overnight. Plasmids containing the different BDNF 5'UTR exons were then transiently transfected into SH-SY5Y cells using Lipofectamine™ 2000 (Invitrogen) at high cell density (70-75%), according to the manufacturer's protocol. 24 hours after transfection, the cells were treated with the listed receptor agonists at the following concentrations: KCL (50mM), BDNF (50ng/ml), Glutammate (GLU: 20µM), S)-3,5-Dihydroxyphenylglycine (DHPG: 50µM), AMPA (30µM), NMDA (30µM), Acetylcholine (AcH: 30µM), Norepinephrine (NE: 50µM), Dopamine (DOPA: 40 µM) and Serotonin (5-HT 50µM). All agonists were purchased from Sigma Aldrich with the exception of DHPG, AMPA and NMDA that were from Ascent Scientific. Then dual luciferase assay was performed as described according to the manufacturer's instructions (Promega).

### MTT Viability Assay

Each drug was also tested in a viability assay to demonstrate that the concentration used was not toxic for the cells as no appreciable cell death could be detected. Using a 96-well plate 100µl of SHSY-5Y cells were distributed per well, maintaining them overnight at 37ªC in a 5% CO₂-humified incubator. On the following day the cells were incubated for 3h at 37ºC with the following drugs:

KCI (50mM), BDNF (50ng/ml), Glutammate (GLU: 20µM), S)-3,5-Dihydroxyphenylglycine (DHPG: 50µM), AMPA (30µM), NMDA (30µM), Acetylcholine (AcH: 10µM), Norepinephrine (NE: 50µM), Dopamine (DOPA: 40µM) and Neurotrophin-3 (NT3: 50ng/ml). After that, 20µl of aseptically prepared 5mg/mL MTT was added, adding it also to an additional quadruplicate of wells without cells, as an internal control. The cells were again incubated at 37ºC this time for 2h, allowing the formation of formazan crystals by the mitochondrial dehydrogenase of viable cells. Afterwards, the media was carefully removed, and the formazan crystals were solubilised by adding 200µl of MTT solvent per well, a solution that contains acidified isopropanol (4mM HCl and 0.1% Nondet P-40 in isopropanol). The crystals were further dissolved by gently pipeting the solution up and down being the resulting purple solution spectrophotometrically measured at 570nm. An increased absorbance can therefore be directly correlated to the cell number, allowing one to determine the cellular viability after a specific treatment. Since the method is dependent on the physiological state of the cells, the assay was always performed after checking the cellular morphology prior treatment. Moreover, two controls were performed: (1) untreated cells, an internal control to verify the drug-dependent effect; and (2) KCI 200mM treated cells, in which a cytotoxic concentration was used, thus allowing us to verify the assay fidelity.

### Results

Figure 3 shows histograms in which the response to KCI, BDNF, Glutammate, (S)-3,5-Dihydroxyphenylglycine (DHPG), AMPA, NMDA, Acetylcholine, Norepinephrine, Dopamine and 5-HT, measured in RLU for each splice variant (5'UTR) is shown. Data are expressed as ratio F-luc/R-Luc (triplicate experiments). Error bars = SE. (ANOVA on ranks *=p<0.05). These results show that the different agonists are able to modulate the reporter gene expression in a specific and transcript-dependent way (Figure 3). In particular KCI, BDNF, Glutamate and Norepinephrine stimulation are able to significantly increase the translation of exon1, 2B and 4 -containing transcripts; the expression of exon 3- containing plasmid is only induced by the general KCI stimulation, instead exon 8 seem to be responsive to both BDNF and Glutamate; but only the latter is able to induce the expression of exon 2C containing plasmid. In order to further analyze Glutamate stimulation, the activation of the specific glutamate ionotropic receptors (AMPA and NMDA) and the metabotrophic receptor (DHPG) was investigated: results show that the enhancement of exon2C-containing expression is mediated by NMDA receptor but translation of the other BDNF transcripts is not stimulated by these specific agonists, suggesting the need of multiple glutamate receptor stimulation in order to increase the reporter expression. Norepinephrine specific increased exon2C and exon7 reporter expression. Among the agonists that were assayed in this experiment, Serotonin was the agonist which enhanced the largest number of BDNF splice variants as it resulted able to increase the translation of every BDNF exons-containing plasmid, with the exception of those containing exon 2A, 4 and 8.

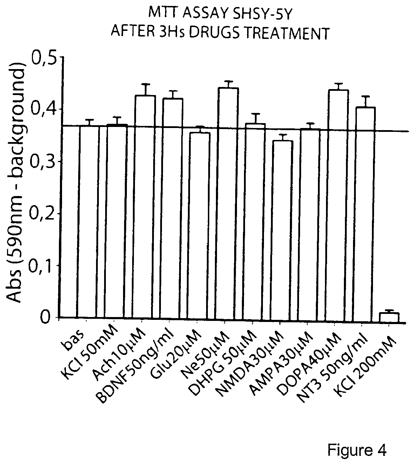

None of the drugs used at the indicated concentrations caused death in SY-SY5Y human neuroblastoma cells (Figure 4). It was observed that Acethylcholine (ACh 10µM), Norepineprhine (NE, 50uM), Dopamine (DOPA, 40µM) have a neurotrophic effect as they promote a small increase in cell survival similar to that seen with the two neurotrophins BDNF and NT3 (both 50 ng/ml).

### EXAMPLE 5

### Response of BDNF CDS and 3'UTR variants to different agonists

Basal and drug-induced translation of a luciferase reporter gene driven by BDNF short and long 3'UTR or coding region (CDS) sequences were measured. One day prior transfection, undifferentiated neuro-blastoma SH-SY5Y cells were seeded into a 96-well white plate with clear bottom (PBI International) at a confluency of 50-60% and cultured overnight. Plasmids containing the BDNF 3'UTR short or long or CDS were then transiently transfected into SH-SY5Y cells using Lipofectamine™ 2000 (Invitrogen) at high cell density (70-75%), according to the manufacturer's protocol. 24 hours after transfection, the cells were treated with the listed inhibitors at the specified concentrations: KCI (50mM), Glutammate (GLU: 20µM), S)-3,5-Dihydroxyphenylglycine (DHPG: 50µM), AMPA (30µM), NMDA (30µM), Acetylcholine (AcH: 30µM), Norepinephrine (NE: 50µM), Dopamine (DOPA: 40µM) and Serotonin (5-HT 50µM).

BDNF (50 ng/µl) was specifically employed for the stimulation of the 3'UTR long containing plasmid and NT3 (50 ng/µl) for 3'UTR short containing plasmid. Then luciferase assay was performed as previously described according to the manufacturer's instructions (Promega).

### Results

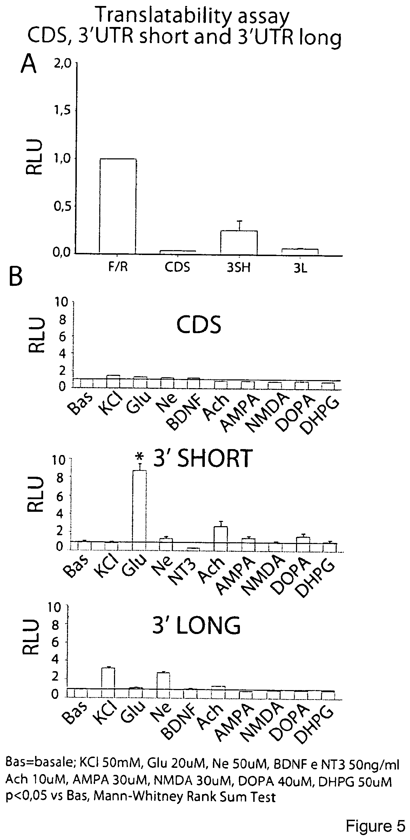

The CDS and the short and long 3'UTR displayed a different profile of responses to drug stimulations as shown in Figure 5. In particular, BDNF CDS resulted to have no regulatory effect on translation, while the short 3'UTR promoted translation more efficiently than the long 3'UTR (Figure 5A). In another set of experiments in transfected SHSY-5Y (Figure 5B), we show that the translation of the CDS and 3'UTR-long transcripts is not enhanced after treatment with the different receptor agonists, even if there is a slightly but not significant increase in 3'UTR-long-containing transcripts translation after the general KCI stimulation. In contrast, there is a striking increase in 3'UTR-short-containing transcripts translation after glutamate treatment. These results confirm the hypothesis that the BDNF CDS is translated at low levels in basal conditions and that it needs the 5'UTR and 3'UTR regions in order to become sensitive to the different stimuli. It is also important to note that the 3'UTR regions are regulated by specific signals and that the short variant is the most responsive one.

### EXAMPLE 6

### Response of BDNF 5'UTR/3'UTR constructs to different antagonists

The present invention can be used not only to identify compounds able to increase BDNF translation, but also to screen for potential inhibitors of BDNF protein production. To this aim a series of vectors were constructed which recapitulate the physiological regulation of BDNF translation in that the firefly luciferase gene was flanked both by 5'UTR regions as well as 3'UTR. These constructs included:
17) pN1-exonl-Fluc-3'UTR short
18) pN1-exon1-Fluc-3'UTR long
19) pN1-exon4-Fluc-3'UTR short
20) pN1-exon4-Fluc-3'UTR long
21) pN1-exon6-Fluc-3'UTR short
22) pN1-exon6-Fluc-3'UTR long.

Inhibitors of 5'UTR regulation are: rapamycin (rap) = mTOR inhibitor (Takei, Inamura et al. 2004); U0126 = MEK inhibitor (Kanhema, Dagestad et al. 2006); GF = PKC inhibitor (Heikkila, Jalava et al. 1993).

Inhibitors of 3'UTR regulation: KN62 = CaMKII inhibitor (Lu, Hidaka et al. 1996); AuA= Aurora A inhibitor (Soncini, Carpinelli et al. 2006) and PP2 = Src tyrosine kinase inhibitor (Perkinton, Sihra et al. 1999).

Aurora kinase A inhibitor (PHA-680632) was a gift from Nerviano Medical Sciences S.r.l. (Milan, Italy); PP2 (1-tert-butyl-3-(4-chlorophenyl)-1 H-pyrazolo[3-4] pyrimidin-4-amine), rapamycin was purchased from Ascent Scientific (Bristol, UK), Bisindolylmaleimide I (2-[1-(3-dimethylaminopropyl)-1 H-indol-3-yl]-3-(1 H-indol-3yl)-maleimide, GF 109203X, Gö 6850), KN62 (1-[N,O-bis-5-isoquinolinesulfonyl)-N-methyl-L-tyrosyl]-4-phenylpiperazine), U0126 (1,4-diamino-2,3-dicyano-1,4-bis(2-aminophenylthio)butadiene), and U0124 (1,4-Diamino-2,3-dicyano-1,4-bis(methylthio) butadiene) from Calbiochem (Damstadt, Germany).

### Results

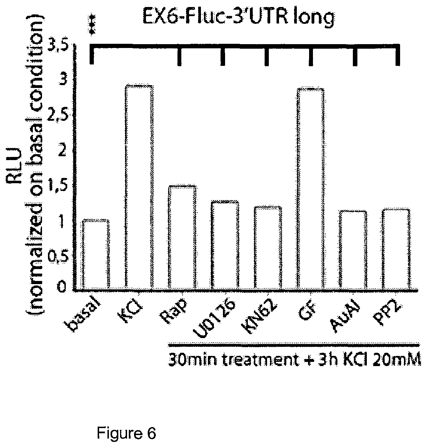

Figure 6 shows the effects of several inhibitors of the signalling pathways which control KCI-induced protein synthesis of the construct pN1-exon6-Fluc-3'UTR long construct. Relative luciferase units (RLU) describe in absolute values for the Fluc/Rluc (F/R) ratio obtained from each condition - basal, 3h of 20mM KCI, 30min of treatment - rapamycin 20nM, U0126 50µM, KN62 20µM, GF 50nM, Aurora A inhibitor 10µM or PP2 20µM, prior 3h of depolarization. A value of 1.0 was assumed for the basal condition. The represented data (mean + standard error) corresponds to three independent experiments in quadruplicate ***, P<0.001; **, P<0.01; *, P<0.05, One Way Anova followed by a multiple comparison versus Fluc construct procedure with Holm-Sidak's method.

We show that 30min of pre-treatment with rapamycin 20nM, U0126 50µM, KN62 20µM, Aurora A inhibitor 10µM or PP2 20µM, but not GF 50nM, completely prevents the three-fold increase in translation observed at the end of 3h depolarization with 20mM KCI.

From the above description and the above-noted examples, the advantage attained by the product described and obtained according to the present invention are apparent.

### REFERENCES

Aid, T., A. Kazantseva, et al. (2007). "Mouse and rat BDNF gene structure and expression revisited." J Neurosci Res 85(3): 525-35.
Aliaga, E. E., I. Mendoza, et al. (2009). "Distinct subcellular localization of BDNF transcripts in cultured hypothalamic neurons and modification by neuronal activation." J Neural Transm 116(1): 23-32.
Chen, W. G., Q. Chang, et al. (2003). "Derepression of BDNF transcription involves calcium-dependent phosphorylation of MeCP2." Science 302(5646): 885-9.
Chiaruttini, C., M. Sonego, et al. (2008). "BDNF mRNA splice variants display activity-dependent targeting to distinct hippocampal laminae." Mol Cell Neurosci 37(1): 11-9.
Garzon, D., G. Yu, et al. (2002). "A new brain-derived neurotrophic factor transcript and decrease in brain-derived neurotrophic factor transcripts 1, 2 and 3 in Alzheimer's disease parietal cortex." J Neurochem 82(5): 1058-64.
Heikkila, J., A. Jalava, et al. (1993). "The selective protein kinase C inhibitor GF 109203X inhibits phorbol ester-induced morphological and functional differentiation of SH-SY5Y human neuroblastoma cells." Biochem Biophys Res Commun 197(3): 1185-93.
Huang, E. J. and L. F. Reichardt (2001). "Neurotrophins: roles in neuronal development and function." Annu Rev Neurosci 24: 677-736.
Jiang, X., J. Zhou, et al. (2009). "Human BDNF isoforms are differentially expressed in cocaine addicts and are sorted to the regulated secretory pathway independent of the Met66 substitution." Neuromolecular Med 11(1): 1-12.
Kanhema, T., G. Dagestad, et al. (2006). "Dual regulation of translation initiation and peptide chain elongation during BDNF-induced LTP in vivo: evidence for compartment-specific translation control." J Neurochem 99(5): 1328-37.
Lu, Y. T., H. Hidaka, et al. (1996). "Characterization of a calcium/calmodulin-dependent protein kinase homolog from maize roots showing light-regulated gravitropism." Planta 199(1): 18-24.
Pattabiraman, P. P., D. Tropea, et al. (2005). "Neuronal activity regulates the developmental expression and subcellular localization of cortical BDNF mRNA isoforms in vivo." Mol Cell Neurosci 28(3): 556-70.
Perkinton, M. S., T. S. Sihra, et al. (1999). "Ca(2+)-permeable AMPA receptors induce phosphorylation of cAMP response element-binding protein through a phosphatidylinositol 3-kinase-dependent stimulation of the mitogen-activated protein kinase signaling cascade in neurons." J Neurosci 19(14): 5861-74.
Pruunsild, P., A. Kazantseva, et al. (2007). "Dissecting the human BDNF locus: bidirectional transcription, complex splicing, and multiple promoters." Genomics 90(3): 397-406.
Roth, T. L., F. D. Lubin, et al. (2009). "Lasting epigenetic influence of early-life adversity on the BDNF gene." Biol Psychiatry 65(9): 760-9.
Sakata, K., N. H. Woo, et al. (2009). "Critical role of promoter IV-driven BDNF transcription in GABAergic transmission and synaptic plasticity in the prefrontal cortex." Proc Natl Acad Sci U S A 106(14): 5942-7.
Soncini, C., P. Carpinelli, et al. (2006). "PHA-680632, a novel Aurora kinase inhibitor with potent antitumoral activity." Clin Cancer Res 12(13): 4080-9.
Takei, N., N. Inamura, et al. (2004). "Brain-derived neurotrophic factor induces mammalian target of rapamycin-dependent local activation of translation machinery and protein synthesis in neuronal dendrites." J Neurosci 24(44): 9760-9.
Tongiorgi, E. and G. Baj (2008). "Functions and mechanisms of BDNF mRNA trafficking." Novartis Found Sump 289: 136-47; discussion 147-51, 193-5.
Tongiorgi, E. (2008). "Activity-dependent expression of brain-derived neurotrophic factor in dendrites: facts and open questions." Neurosci Res. 61(4):335-46
Zuccato, C., D. Liber, et al. (2005). "Progressive loss of BDNF in a mouse model of Huntington's disease and rescue by BDNF delivery." Pharmacol Res 52(2): 133-9.

## Claims

1. A method of screening for modulators of the translation of brain-derived neurotrophic factor (BDNF) comprising the steps of:
a) transfecting a mammalian cell with a nucleic acid construct, said nucleic acid construct comprising:
- a 5' untranslated (5'UTR) exon of a mammalian BDNF gene and a 3' untranslated (3'UTR) exon of a mammalian BDNF directly linked to the cDNA of a functional reporter gene, in the absence of any other BDNF promoter sequence;
b) contacting said mammalian cell with a screening compound;
c) detecting the luminescence produced by said mammalian cell of step b);
d) comparing the luminescence measurements of the cell contacted with the screening compound with the luminescence measurements of a control;
wherein a change in luminescence compared to the control allows to identify a modulator of BDNF translation.

2. The method according to claim 1, wherein said 5' untranslated (5'UTR) exon of a mammalian BDNF gene of step a), is the 5' untranslated (5'UTR) exon of the rat BDNF gene.

3. The method according to claim 2, wherein said 5' untranslated (5'UTR) exon of the rat BDNF gene is selected from the group consisting of SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9 or SEQ ID NO. 10.

4. The method according to anyone of claim 1, wherein said 3' untranslated (3'UTR) exon of a mammalian BDNF is the 3' untranslated (3'UTR) exon of rat BDNF gene.

5. The method according to claim 4, wherein said 3' untranslated (3'UTR) exon of the rat BDNF gene is selected from the group consisting of SEQ ID NO. 13 and SEQ ID NO. 14.

6. The method according to anyone of claims 1 to 5, wherein said mammalian cell grows in adhesion.

7. The method according to claim 6, wherein said mammalian cell is chosen from the group consisting of SH-SY5Y cells, SK-N-SH cells, SK-N-BE cells, NIH-3T3 cells, HeLa cells, HEK 293 cells, and CHO cells.

8. The method according to anyone of claims 1 to 5, wherein said screening compound is a BDNF-translation agonist.

9. The method according to claim 8, wherein said screening compound is selected from the group consisting of ion-channels agonists, neurotransmitters synthesis, release and re-uptake agonsits, neurotransmitter receptors agonists, growth-factors receptors agonists, translation factors agonists and intracellular signalling agonists.

10. The method according to anyone of claims 1 to 5, wherein said screening compound is a BDNF-translation antagonist.

11. The method according to claim 10, wherein said screening compound is selected from the group consisting of ion-channels antagonists, neurotransmitters synthesis, release and re-uptake antagonists, neurotransmitter receptors antagonists, growth-factors receptors antagonists, translation factors antagonists and intracellular signalling antagonists.

12. The method according to anyone of claims 1 to 11, wherein said reporter gene is a luciferase gene.

13. The method according to claim 12, wherein said luciferase gene is chosen from the group consisting of Firefly luciferase and Renilla luciferase.

14. The method according to anyone of claims 1 to 13, wherein said method of screening is carried out by the high throughput screening (HTS) technique.

15. A nucleic acid construct comprising:
a 5' untranslated (5'UTR) exon of the rat BDNF gene selected from the group consisting of SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9 or SEQ ID NO. 10 directly linked to the cDNA of a functional reporter gene, in the absence of any other BDNF promoter sequence; and
a 3' untranslated (3'UTR) exon of the rat BDNF gene selected from the group consisting of SEQ ID NO. 13 and SEQ ID NO. 14.

16. The nucleic acid construct according to claim 15, wherein said reporter gene is a luciferase gene.

17. The nucleic acid construct according to claim 16, wherein said luciferase gene is chosen from the group consisting of Firefly luciferase and Renilla luciferase.

18. A plasmid comprising the nucleic acid construct according to claims 15, 16 or 17.

## Patentansprüche

1. Verfahren zum Screening auf Modulatoren der Translation von vom Gehirn stammendem neurotrophem Faktor (BDNF), umfassend die Schritte:
a) Transfizieren einer Säugetierzelle mit einem Nucleinsäurekonstrukt, wobei das Nucleinsäurekonstrukt umfasst:
- ein 5'-untranslatiertes (5'UTR) Exon eines Säugetier-BDNF-Gens und ein 3'-untranslatiertes (3'UTR) Exon eines Säugetier-BDNF, direkt verknüpft mit der cDNA eines funktionellen Reporter-Gens, in Abwesenheit einer anderen BDNF-Promotorsequenz;
b) In-Kontakt-Bringen der Säugetierzelle mit einer Screening-Verbindung;
c) Detektieren der Lumineszenz, die durch die Säugetierzelle von Schritt b) produziert wird;
d) Vergleichen der Lumineszenzmessungen der Zelle, die mit der Screening-Verbindung in Kontakt gebracht wurde, mit den Lumineszenzmessungen einer Kontrolle;
wobei eine Änderung der Lumineszenz im Vergleich zu der Kontrolle es erlaubt, einen Modulator der BDNF-Translation zu identifizieren.

2. Verfahren nach Anspruch 1, wobei das 5'-untranslatierte (5'UTR) Exon eines Säugetier-BDNF-Gens von Schritt a) das 5'-untranslatierte (5'UTR) Exon des Ratten-BDNF-Gens ist.

3. Verfahren nach Anspruch 2, wobei das 5'-untranslatierte (5'UTR) Exon des Ratten-BDNF-Gens aus der Gruppe, bestehend aus SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9 oder SEQ ID NO. 10, ausgewählt wird.

4. Verfahren nach Anspruch 1, wobei das 3'-untranslatierte (3'UTR) Exon des Säugetier-BDNF das 3'untranslatierte (3'UTR) Exon von Ratten-BDNF-Gen ist.

5. Verfahren nach Anspruch 4, wobei das 3'-untranslatierte (3'UTR) Exon des Ratten-BDNF-Gens aus der Gruppe, bestehend aus SEQ ID NO. 13 und SEQ ID NO. 14, ausgewählt wird.

6. Verfahren nach einem der Ansprüche 1 bis 5, wobei die Säugetierzelle in Adhäsion wächst.

7. Verfahren nach Anspruch 6, wobei die Säugetierzelle aus der Gruppe, bestehend aus SH-SY5Y-Zellen, SK-N-SH-Zellen, SK-N-BE-Zellen, NIH-3T3-Zellen, HeLa-Zellen, HEK 293-Zellen und CHO-Zellen, ausgewählt wird.

8. Verfahren nach einem der Ansprüche 1 bis 5, wobei die Screening-Verbindung ein BDNF-Translations-Agonist ist.

9. Verfahren nach Anspruch 8, wobei die Screening-Verbindung aus der Gruppe, bestehend aus lonenkanal-Agonisten, Neurotransmitter-Synthese, Freisetzungs- und Wiederaufnahme-Agonisten, Neurotransmittel-Rezeptor-Agonisten, Wachstumsfaktor-Rezeptor-Agonisten und intrazellulären Signalübertragungs-Agonisten, ausgewählt wird.

10. Verfahren nach einem der Ansprüche 1 bis 5, wobei die Screening-Verbindung ein BDNF-Translations-Antagonist ist.

11. Verfahren nach Anspruch 10, wobei die Screening-Verbindung aus der Gruppe, bestehend aus lonenkanal-Antagonisten, Neurotransmitter-Synthese, Frei-setzungs- und Wiederaufnahme-Antagonisten, Neurotransmitter-Rezeptor-Antagonisten, Wachstumsfaktor-Rezeptor-Antagonisten, Translationsfaktor-Antagonisten und intrazellulären Signalübertragungs-Antagonisten, ausgewählt wird.

12. Verfahren nach einem der Ansprüche 1 bis 11, wobei das Reportergen ein Luciferase-Gen ist.

13. Verfahren nach Anspruch 12, wobei das Luciferase-Gen aus der Gruppe, bestehend aus Glühwürmchen-Luciferase und Renilla-Luciferase, ausgewählt wird.

14. Verfahren nach einem der Ansprüche 1 bis 13, wobei das Screening-Verfahren mittels der Hochdurchsatz-Screening-Technik (high throughput screen-ing, HTS) durchgeführt wird.

15. Nucleinsäurekonstrukt, umfassend:
ein 5'-untranslatiertes (5'UTR) Exon des Ratten-BDNF-Gens, ausgewählt aus der Gruppe, bestehend aus SEQ ID NO. 1, SEQ ID NO. 2, SEQ ID NO. 3, SEQ ID NO. 4, SEQ ID NO: 5, SEQ ID NO. 6, SEQ ID NO. 7, SEQ ID NO. 8, SEQ ID NO. 9 oder SEQ ID NO. 10, direkt verknüpft mit der cDNA eines funktionellen Reportergens, in Abwesenheit einer anderen BDNF-Promotorsequenz, und ein 3'-untranslatiertes (3'UR) Exon des Ratten-BDNF-Gens, ausgewählt aus der Gruppe, bestehend aus SEQ ID NO. 13 und SEQ ID NO. 14.

16. Nucleinsäurekonstrukt nach Anspruch 15, wobei das Reportergen ein Luciferase-Gen ist.

17. Nucleinsäurekonstukt nach Anspruch 16, wobei das Luciferase-Gen aus der Gruppe, bestehend aus Glühwürmchen-Luciferase und Renilla-Luciferase, ausgewählt ist.

18. Plasmid, umfassend das Nucleinsäurekonstrukt nach Anspruch 15, 16 oder 17.

## Revendications

1. Procédé de criblage pour des modulateurs de la traduction du facteur neurotrophique dérivé du cerveau (BDNF) comprenant les étapes suivantes :
a) la transfection d'une cellule de mammifère avec une construction d'acide nucléique, ladite construction d'acide nucléique comprenant :
- un exon non traduit en 5' (5'UTR) d'un gène de BDNF de mammifère et un exon non traduit en 3' (3'UTR) d'un BDNF de mammifère liés directement à l'ADNc d'un gène rapporteur fonctionnel, en l'absence de toute autre séquence promoteur du BDNF ;
b) la mise en contact de ladite cellule de mammifère avec un composé de criblage ;
c) la détection de la luminescence produite par ladite cellule de mammifère de l'étape b) ;
d) la comparaison des mesures de luminescence de la cellule mise en contact avec le composé de criblage aux mesures de luminescence d'un témoin ;
dans lequel un changement de luminescence comparativement au témoin permet d'identifier un modulateur de la traduction du BDNF.

2. Procédé selon la revendication 1, dans lequel ledit exon non traduit en 5' (5'UTR) d'un gène de BDNF de mammifère de l'étape a), est l'exon non traduit en 5' (5'UTR) du gène du BDNF du rat.

3. Procédé selon la revendication 2, dans lequel ledit exon non traduit en 5' (5'UTR) du gène du BDNF du rat est choisi dans le groupe constitué de SEQ ID NO _{:} 1, SEQ ID NO _{:} 2, SEQ ID NO _{:} 3, SEQ ID NO _{:} 4, SEQ ID NO _{:} 5, SEQ ID NO _{:} 6, SEQ ID NO _{:} 7, SEQ ID NO _{:} 8, SEQ ID NO _{:} 9 ou SEQ ID NO _{:} 10.

4. Procédé selon la revendication 1, dans lequel ledit exon non traduit en 3' (3'UTR) d'un BDNF de mammifère est l'exon non traduit en 3' (3'UTR) du gène du BDNF du rat.

5. Procédé selon la revendication 4, dans lequel ledit exon non traduit en 3' (3'UTR) du gène du BDNF du rat est choisi dans le groupe constitué de SEQ ID NO _{:} 13 et SEQ ID NO _{:} 14.

6. Procédé selon l'une quelconque des revendications 1 à 5, dans lequel ladite cellule de mammifère se développe en adhérence.

7. Procédé selon la revendication 6, dans lequel ladite cellule de mammifère est choisie dans le groupe constitué des cellules SH-SY5Y, les cellules SK-N-SH, les cellules SK-N-BE, les cellules NIH-3T3, les cellules HeLa, les cellules HEK 293, et les cellules CHO.

8. Procédé selon l'une quelconque des revendications 1 à 5, dans lequel ledit composé de criblage est un agoniste de la traduction du BDNF.

9. Procédé selon la revendication 8, dans lequel ledit composé de criblage est choisi dans le groupe constitué des agonistes des canaux ioniques, des agonistes de la synthèse, de la libération et de la recapture des neurotransmetteurs, des agonistes des récepteurs des facteurs de croissance, des agonistes des facteurs de la traduction et des agonistes de la signalisation intracellulaire.

10. Procédé selon l'une quelconque des revendications 1 à 5, dans lequel ledit composé de criblage est un antagoniste de la traduction du BDNF.

11. Procédé selon la revendication 10, dans lequel ledit composé de criblage est choisi dans le groupe constitué des antagonistes des canaux ioniques, des antagonistes de la synthèse, de la libération et de la recapture des neurotransmetteurs, des antagonistes des récepteurs des neurotransmetteurs, des antagonistes des récepteurs des facteurs de croissance, des antagonistes des facteurs de la traduction et des antagonistes de la signalisation intracellulaire.

12. Procédé selon l'une quelconque des revendications 1 à 11, dans lequel ledit gène rapporteur est un gène de la luciférase.

13. Procédé selon la revendication 12, dans lequel ledit gène de la luciférase est choisi dans le groupe constitué de la luciférase de luciole et de la luciférase de Renilla.

14. Procédé selon l'une quelconque des revendications 1 à 13, dans lequel ledit procédé de criblage est réalisé par la technique de criblage à haut débit (HTS).

15. Construction d'acide nucléique comprenant :
un exon non traduit en 5' (5'UTR) du gène du BDNF du rat choisi dans le groupe constitué de SEQ ID NO _{:} 1, SEQ ID NO _{:} 2, SEQ ID NO _{:} 3, SEQ ID NO _{:} 4, SEQ ID NO _{:} 5, SEQ ID NO _{:} 6, SEQ ID NO _{:} 7, SEQ ID NO _{:} 8, SEQ ID NO _{:} 9 ou SEQ ID NO _{:} 10 lié directement à l'ADNc d'un gène rapporteur fonctionnel, en l'absence de toute autre séquence promoteur du BDNF ; et
un exon non traduit en 3' (3'UTR) du gène du BDNF du rat choisi dans le groupe constitué de SEQ ID NO _{:} 13 et SEQ ID NO _{:} 14.

16. Construction d'acide nucléique selon la revendication 15, dans laquelle ledit gène rapporteur est un gène de la luciférase.

17. Construction d'acide nucléique selon la revendication 16, dans laquelle ledit gène de la luciférase est choisi dans le groupe constitué de la luciférase de luciole et de la luciférase de Renilla.

18. Plasmide comprenant la construction d'acide nucléique selon les revendications 15, 16 ou 17.
